# EUROPEAN PATENT APPLICATION

(11) **EP 4 238 655 A1**
(43) Date of publication of application: **06.09.2023**
(21) Application number: 23158400.4
(22) Date of filing: 24.02.2023
(51) Int. Cl.: B04B 5/04, B04B 13/00

(54) **CONTINUOUS-FLOW CENTRIFUGE CHAMBERS HAVING A NON-UNIFORM RADIUS HIGH-G WALL**

(30) Priority: 28.02.2022 US 202263314787 P
(71) Applicant: Fenwal, Inc., Lake Zurich, IL 60047 (US)
(72) Inventor: Kusters, Benjamin E., Lake Zurich, 60047 (US); Brown, Richard I., Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

Fluid separation chambers are provided with a central hub, with generally annular low-G and high-G walls (58, 60a) extending about the hub to define therebetween a separation channel (66b). A plurality of radial walls (70, 72) extend from the hub to the channel to define an inlet passage (68), two outlet passages (74, 76), and a terminal wall separating an upstream end (67) of the separation channel from a downstream end (69) of the channel. The radius of the high-G wall is greater at the downstream end of the separation channel than at the upstream end, which may include the radius gradually increasing along a tapered section (92) of the high-G wall. The tapered section may extend from the upstream end of the separation channel to the downstream end of the channel or along a smaller length of the channel. The radius of the low-G wall may similarly increase from the upstream end of the separation channel to the downstream end.

## Description

### Background

### Field of the Disclosure

The present disclosure relates to continuous-flow centrifuges. More particularly, the present disclosure relates to continuous-flow centrifuge chambers having a high-G wall with a non-uniform radius.

### Description of Related Art

Various blood processing systems now make it possible to collect particular blood constituents, rather than whole blood, from a blood source. Typically, in such systems, whole blood is drawn from a source, the particular blood component or constituent is removed and collected, and the remaining blood constituents are returned to the source.

Whole blood is typically separated into its constituents through centrifugation. This requires that the whole blood be passed through a centrifuge after it is withdrawn from, and before it is returned to, the source. To avoid contamination and possible infection of the source, the blood is preferably contained within a sealed, sterile fluid flow circuit during the entire centrifugation process. Typical blood processing systems thus include a permanent, reusable centrifuge assembly containing the hardware (drive system, pumps, valve actuators, programmable controller, and the like) that spins and pumps the blood, and a disposable, sealed and sterile fluid flow circuit that is mounted in cooperation on the hardware. The centrifuge assembly engages and spins a disposable centrifuge chamber of the fluid flow circuit during a collection procedure. The blood, however, makes actual contact only with the fluid flow circuit, which assembly is used only once and then discarded.

As the whole blood is spun by the centrifuge, the heavier (greater specific gravity) components, such as red blood cells, move radially outwardly away from the center of rotation toward the outer or "high-g" wall of the separation chamber. The lighter (lower specific gravity) components, such as plasma, migrate toward the inner or "low-g" wall of the separation chamber. Various ones of these components can be selectively removed from the whole blood by forming appropriately located channeling seals and outlet ports in the separation chamber.

Centrifugation chambers of this type are well-known, with exemplary centrifugation chambers being described in U.S. Patent No. 9,327,296 and PCT Patent Application Publication No. WO 2018/053217 A1, the disclosures of both of which are hereby incorporated herein by reference. While conventional centrifugation chambers have proven to be suitable for separation blood and other biological fluids, it would be advantageous to provide chambers improving on the performance and/or manufacturability of such known chambers.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a fluid separation chamber for rotation about an axis includes a central hub coinciding with the axis. A generally annular low-G wall and a generally annular high-G wall extend about the central hub in a spaced apart relationship to define therebetween a separation channel having an upstream end and a downstream end. A plurality of radial walls extend from the central hub to the separation channel to define a terminal wall separating the upstream end of the separation channel from the downstream end of the separation channel, an inlet passage at the upstream end of the separation channel, and low-G and high-G outlet passages. The high-G wall has a greater radius at the downstream end of the separation channel than at the upstream end of the separation channel at each axial position.

These and other aspects of the present subject matter are set forth in the following detailed description of the accompanying drawings.

### Brief Description of the Drawings

Fig. 1 is a perspective view of an exemplary fluid processing device that comprises a component of a fluid processing system according to an aspect of the present disclosure;
Fig. 2 is a schematic view of an exemplary disposable fluid flow circuit that may be mounted to the fluid processing device of Fig. 1 to complete a fluid processing system according to an aspect of the present disclosure;
Fig. 3 is a perspective view of an exemplary centrifugal separator of the fluid processing device of Fig. 1, with the centrifuge chamber of a fluid flow circuit mounted therein;
Fig. 4 is a top plan view of an exemplary cassette of a fluid flow circuit, which can be actuated to perform a variety of different fluid processing procedures in association with the fluid processing device shown in Fig. 1;
Fig. 5 is a perspective view of the centrifugal separator of Fig. 3, with selected portions thereof broken away to show a light source of an interface monitoring assembly;
Fig. 6 is a perspective view of the centrifugal separator of Fig. 3, with the light source operating to transmit a light beam to a light detector of the interface monitoring assembly;
Fig. 7 is a perspective view of the centrifugal separator of Fig. 3, with selected portions thereof broken away to show the light source and light detector of the interface monitoring assembly;
Fig. 8 is a perspective view of an exemplary centrifuge chamber of the fluid flow circuit of Fig. 2;
Fig. 9 is a perspective view of a conventionally configured fluid flow path or separation channel that may be defined by the centrifuge chamber of Fig. 8;
Fig. 9A is a bottom plan view of the separation channel of Fig. 9;
Fig. 10 is a bottom plan view of a fluid flow path or separation channel that may be defined by the centrifuge chamber of Fig. 8, according to an aspect of the present disclosure;
Fig. 10A illustrates the separation channel of Fig. 10 overlaid onto the separation channel of Fig. 9A;
Fig. 11 is an enlarged perspective view of a portion of a channel of the centrifuge chamber of Fig. 8, with an interface between separated fluid components being positioned at a (typically) desired location on a ramp defined within the channel;
Fig. 12 is an enlarged perspective view of the channel and ramp of Fig. 11, with the interface being at a (typically) undesired high location on the ramp;
Fig. 13 is an enlarged perspective view of the channel and ramp of Fig. 11, with the interface being at a (typically) undesired low location on the ramp;
Fig. 14 is a perspective view of a prismatic reflector used in combination with the centrifuge chamber of Fig. 8; and
Fig. 15 is a perspective view of the prismatic reflector of Fig. 14, showing light being transmitted therethrough.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

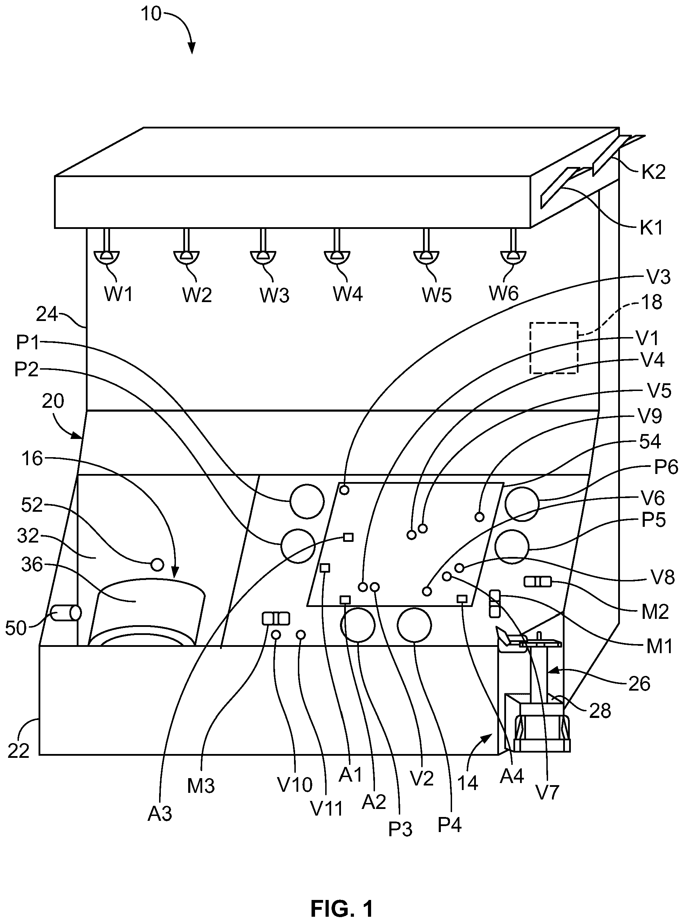

Figs. 1-15 show components of a blood or fluid processing system that embodies various aspects of the present subject matter. While the system may be described herein in terms of its use in separating blood into two or more components, it should be understood that systems according to the present disclosure can be used for processing a variety of biological or bodily fluids (including fluids containing both bodily and non-bodily fluids, such as anticoagulated blood), as well as non-bodily fluids.

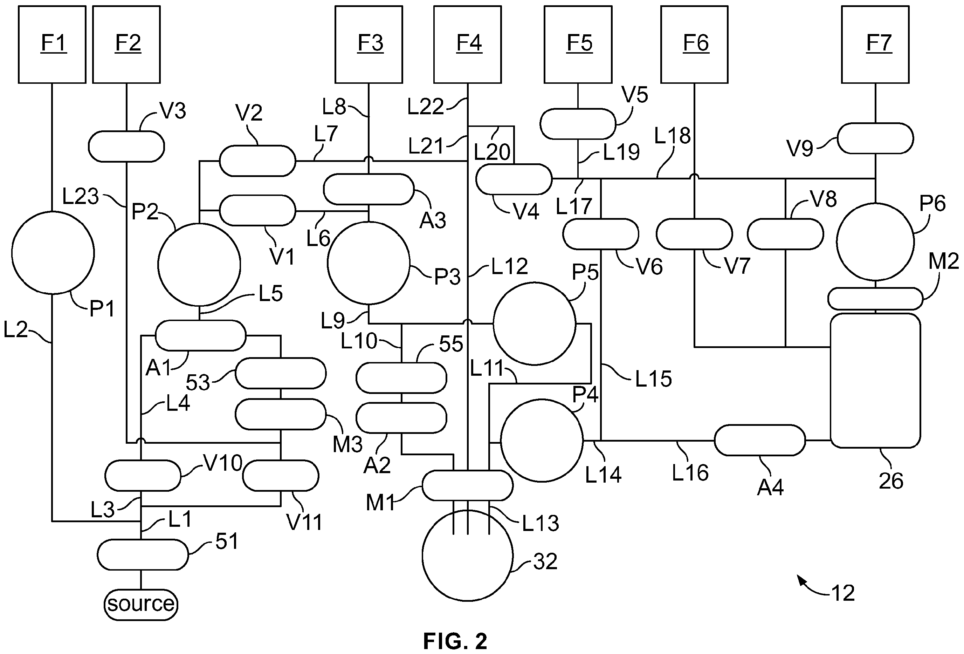

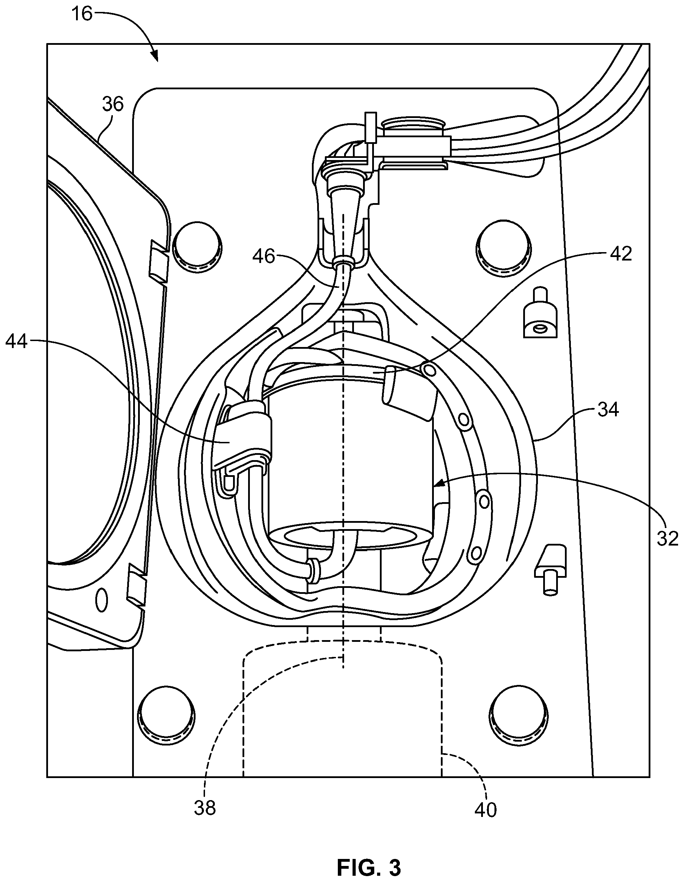

Fluid processing systems according to the present disclosure typically include two principal components, a durable and reusable fluid processing device 10 (Fig. 1) and a disposable fluid flow circuit 12 (Fig. 2). The illustrated fluid processing device 10 includes a spinning membrane separator drive unit 14 (Fig. 1), a centrifuge or centrifugal separator 16 (Fig. 3), additional components that control fluid flow through the disposable flow circuit 12, and a controller 18 (Fig. 1), which governs the operation of the other components of the fluid processing device 10 to perform a procedure selected by the operator. The principles described herein regarding setting the rotation rate of a continuous-flow centrifuge are not limited to any particular fluid processing systems or procedures, so no complete fluid processing devices or procedures will be described in detail herein. However, reference may be made to PCT Patent Application Publication No. WO 2018/053217 A1 for a detailed description of the fluid processing device 10 of Fig. 1, along with various exemplary procedures that may be carried out using such a system.

### I. The Durable Fluid Processing Device

The fluid processing device 10 (Fig. 1) is configured as a durable item that is capable of long-term use. It should be understood that the fluid processing device 10 of Fig. 1 is merely exemplary of one possible configuration and that fluid processing devices according to the present disclosure may be differently configured. For example, it is within the scope of the present disclosure for the fluid processing device to omit a spinning membrane separator drive unit 14.

In the illustrated embodiment, the fluid processing device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24.

### A. Spinning Membrane Separator Drive Unit

The illustrated fluid processing device 10 includes a spinner support or spinning membrane separator drive unit 14 (Fig. 1) for accommodating a generally cylindrical spinning membrane separator 26 of a fluid flow circuit 12 (Fig. 2). U.S. Patent No. 5,194,145 (which is hereby incorporated herein by reference) describes an exemplary spinning membrane separator drive unit that would be suitable for incorporation into the fluid processing device 10, but it should be understood that the spinning membrane separator drive unit 14 may be differently configured without departing from the scope of the present disclosure. The principles described herein are specific to the configuration of a chamber 32 received by the centrifugal separator 16, so the spinning membrane separator drive unit 14 is not described in detail herein.

### B. Centrifugal Separator

The illustrated centrifugal separator 16 includes a centrifuge compartment 34 that may receive the other components of the centrifugal separator 16 (Fig. 3). The centrifuge compartment 34 may include a lid 36 that is opened to insert and remove a centrifuge chamber 32 of the fluid flow circuit 12. During a separation procedure, the lid 36 may be closed with the centrifuge chamber 32 positioned within the centrifuge compartment 34, as the centrifuge chamber 32 is spun or rotated about an axis 38 under the power of an electric drive motor or rotor 40 of the centrifugal separator 16.

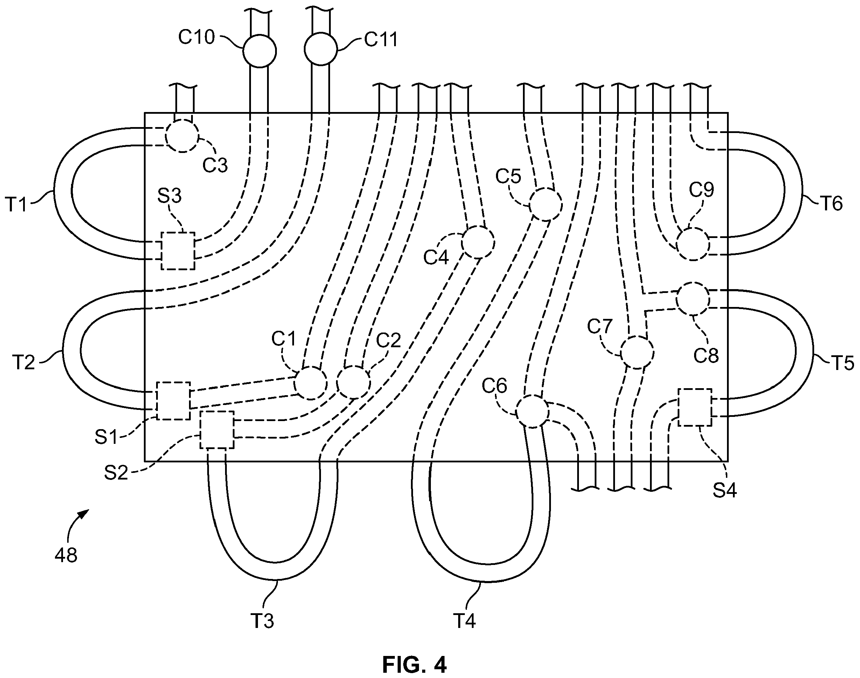

The particular configuration and operation of the centrifugal separator 16 depends upon the particular configuration of the centrifuge chamber 32 of the fluid flow circuit 12. In one embodiment, the centrifugal separator 16 is similar in structure and operation to that of the ALYX^{®} processing device manufactured by Fenwal, Inc. of Lake Zurich, Illinois, which is an affiliate of Fresenius Kabi AG of Bad Homburg, Germany, as described in greater detail in U.S. Patent No. 8,075,468, which is hereby incorporated herein by reference. More particularly, the centrifugal separator 16 may include a carriage or support 42 that holds the centrifuge chamber 32 and a yoke member 44. The yoke member 44 engages an umbilicus 46 of the fluid flow circuit 12, which extends between the centrifuge chamber 32 and a cassette 48 of the fluid flow circuit 12 (Fig. 4). The yoke member 44 causes the umbilicus 46 to orbit around the centrifuge chamber 32 at a one omega rotational speed. The umbilicus 46 twists about its own axis as it orbits around the centrifuge chamber 32. The twisting of the umbilicus 46 about its axis as it rotates at one omega with the yoke member 44 imparts a two omega rotation to the centrifuge chamber 32, according to known design. The relative rotation of the yoke member 44 at a one omega rotational speed and the centrifuge chamber 32 at a two omega rotational speed keeps the umbilicus 46 untwisted, avoiding the need for rotating seals.

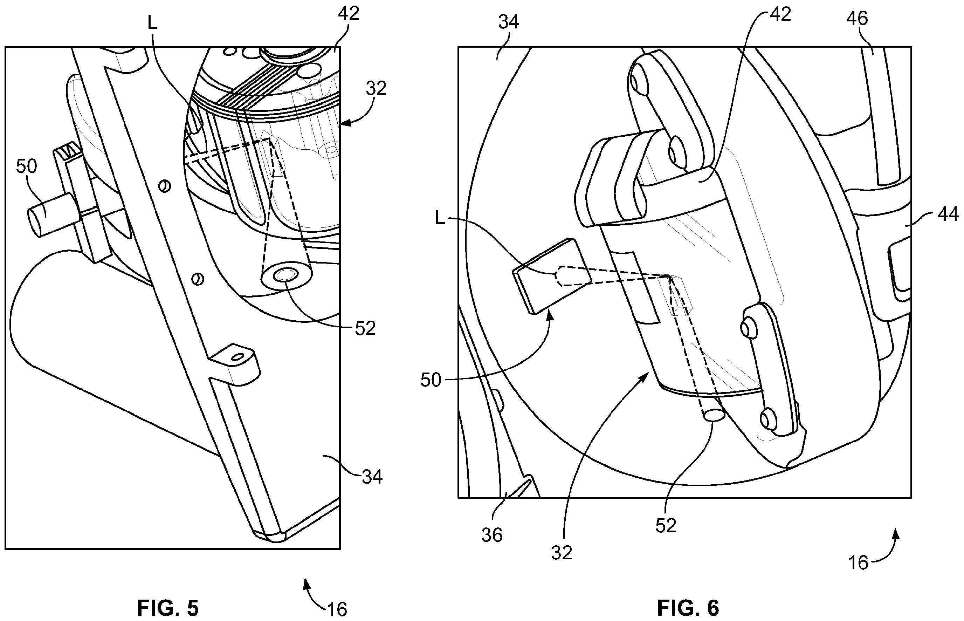

A fluid is introduced into the centrifuge chamber 32 by the umbilicus 46, with the fluid being separated (e.g., into a layer of less dense components, such as platelet-rich plasma, if the fluid is blood, and a layer of more dense components, such as packed red blood cells, if the fluid is blood) within the centrifuge chamber 32 as a result of centrifugal forces as it rotates. Components of an interface monitoring assembly may be positioned within the centrifuge compartment 16 to oversee separation of fluid within the centrifuge chamber 32. As shown in Figs. 5-7, the interface monitoring assembly may include a light source 50 and a light detector 52, which is positioned and oriented to receive at least a portion of the light emitted by the light source 50. The illustrated light source 50 and light detector 52 are associated with stationary surfaces of the centrifuge compartment 34, but either or both may instead be associated with a movable structure or component of the fluid processing device 10, as in U.S. Patent No. 5,316,667, which is hereby incorporated herein by reference.

The orientation of the various components of the interface monitoring system depends at least in part on the particular configuration of the centrifuge chamber 32, which will be described in greater detail herein. In general, though, the light source 50 emits a light beam "L" (e.g., a laser light beam) through the separated fluid components within the centrifuge chamber 32 (which may be formed of a material that substantially transmits the light L or at least a particular wavelength of the light L without absorbing it). A portion of the light L reaches the light detector 52, which transmits a signal to the controller 18 that is indicative of the location of an interface between the separated fluid components. If the controller 18 determines that the interface is in the wrong location (which can affect the separation efficiency of the centrifugal separator 16 and/or the quality of the separated blood components), then it can issue commands to the appropriate components of the fluid processing device 10 to modify their operation so as to move the interface to the proper location.

### C. Other Components Of The Fluid Processing Device

In addition to the spinning membrane separator drive unit 14 and the centrifugal separator 16, the fluid processing device 10 may include other components compactly arranged to aid fluid processing.

The generally horizontal portion 22 of the case 20 of the illustrated fluid processing device 10 includes a cassette station 54, which accommodates a cassette 48 of the fluid flow circuit 12 (Fig. 4). In one embodiment, the cassette station 54 is similarly configured to the cassette station of U.S. Patent No. 5,868,696 (which is incorporated herein by reference), but is adapted to include additional components and functionality. The illustrated cassette station 54 includes a plurality of clamps or valves V1-V9 (Fig. 1), which move between a plurality of positions (e.g., between a retracted or lowered position and an actuated or raised position) to selectively contact or otherwise interact with corresponding valve stations C1-C9 of the cassette 48 of the fluid flow circuit 12 (Figs. 2 and 4). Depending on the configuration of the fluid flow circuit 12, its cassette 48 may not include a valve station C1-C9 for each valve V1-V9 of the cassette station 54, in which case fewer than all of the valves V1-V9 will be used in a separation procedure.

In the actuated position, a valve V1-V9 engages the associated valve station C1-C9 to prevent fluid flow through that valve station C1-C9 (e.g., by closing one or more ports associated with the valve station C1-C9, thereby preventing fluid flow through that port or ports). In the retracted position, a valve V1-V9 is disengaged from the associated valve station C1-C9 (or less forcefully contacts the associated valve station C1-C9 than when in the actuated position) to allow fluid flow through that valve station C1-C9 (e.g., by opening one or more ports associated with the valve station C1-C9, thereby allowing fluid flow through that port or ports). Additional clamps or valves V10 and V11 may be positioned outside of the cassette station 54 to interact with portions or valve stations C10 and C11 (which may be lengths of tubing) of the fluid flow circuit 12 to selectively allow and prevent fluid flow therethrough. The valves V1-V9 and corresponding valve stations C1-C9 of the cassette station 54 and cassette 48 may be differently configured and operate differently from the valves V10 and V11 and valve stations C10 and C11 that are spaced away from the cassette station 54.

The cassette station 54 may be provided with additional components, such as pressure sensors A1-A4, which interact with sensor stations S1-S4 of the cassette 48 to monitor the pressure at various locations of the fluid flow circuit 12. For example, if the fluid source is a human donor, one or more of the pressure sensors A1-A4 may be configured to monitor the pressure of the donor's vein during blood draw and return. Other pressure sensors A1-A4 may monitor the pressure of the spinning membrane separator 26 and the centrifuge chamber 32. The controller 18 may receive signals from the pressure sensor A1-A4 that are indicative of the pressure within the fluid flow circuit 12 and, if a signal indicates a low- or highpressure condition, the controller 18 may initiate an alarm or error condition to alert an operator to the condition and/or to attempt to bring the pressure to an acceptable level without operator intervention.

The fluid processing device 10 may also include a plurality of pumps P1-P6 (which may be collectively referred to as a pump system) to cause fluid to flow through the fluid flow circuit 12. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696. Each pump P1-P6 engages a different tubing loop T1-T6 extending from a side surface of the cassette 48 (Fig. 4) and may be selectively operated under command of the controller 18 to cause fluid to flow through a portion of the fluid flow circuit 12, as will be described in greater detail. In one embodiment, all or a portion of the cassette station 54 may be capable of translational motion in and out of the case 20 to allow for automatic loading of the tubing loops T1-T6 into the associated pump P1-P6.

The illustrated fluid processing device 10 also includes an optical detection assembly or centrifugal separator sensor M1 for determining one or more properties of fluids flowing out of and/or into the centrifugal separator 16. If the fluid flowing out of the centrifugal separator 16 includes red blood cells, the centrifugal separator sensor M1 may be configured to determine the hematocrit of the fluid. If the fluid flowing out of the centrifugal separator 16 is platelet-rich plasma, the centrifugal separator sensor M1 may be configured to determine the platelet concentration of the platelet-rich plasma. The centrifugal separator sensor M1 may detect the one or more properties of a fluid by optically monitoring the fluid as it flows through tubing of the fluid flow circuit 12 or by any other suitable approach. The controller 18 may receive signals from the centrifugal separator sensor M1 that are indicative of the nature of flow into and out of the centrifuge separator 16 (e.g., whether air or a liquid flow is flowing through an inlet or outlet conduit connected to the centrifuge chamber 32, whether fluid is flowing through such conduit or is stagnant, etc.) and use the signals to optimize the separation procedure. If one or more properties of a fluid flowing into or out of the centrifuge chamber 32 is outside of an acceptable range, then the controller 18 may initiate an alarm or error condition to alert an operator to the condition. Exemplary optical detection assemblies are described in U.S. Patent No. 6,419,822 and U.S. Patent Application Publication No. 2019/0369008 (both of which are hereby incorporated herein by reference), but it should be understood that a different approach may also be employed for optically monitoring fluid flow into and out of the centrifugal separator 16.

The illustrated fluid processing device 10 further includes a spinner outlet sensor M2, which accommodates tubing of the fluid flow circuit 12 that flows a separated substance out of the spinning membrane separator 26. The spinner outlet sensor M2 monitors the substance to determine one or more properties of the substance, and may do so by optically monitoring the substance as it flows through the tubing or by any other suitable approach. In one embodiment, separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is incorporated herein by reference) or by any other suitable device and/or method.

The illustrated fluid processing device 10 also includes an air detector M3 (e.g., an ultrasonic bubble detector), which accommodates tubing of the fluid flow circuit 12 that flows fluid to a recipient. It may be advantageous to prevent air from reaching the recipient, so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

The generally vertical portion 24 of the case 18 may include a plurality of weight scales W1-W6 (six are shown, but more or fewer may be provided), each of which may support one or more fluid containers F1-F7 of the fluid flow circuit 12 (Fig. 2). The containers F1-F7 receive fluid components or waste products separated during processing or assorted non-biological fluids (e.g., priming fluids, intravenous fluids, or additive fluids). Each weight scale W1-W6 transmits to the controller 18 a signal that is indicative of the weight of the fluid within the associated container F1-F7 to track the change of weight during the course of a procedure. This allows the controller 18 to process the incremental weight changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports K1 and K2 that may support various components of the fluid flow circuit 12 or other suitably sized and configured objects.

### D. Controller

As described above, the fluid processing device 10 includes a controller 18, which is suitably configured and/or programmed to control operation of the fluid processing device 10. In one embodiment, the controller 18 comprises a main processing unit (MPU), which can comprise, e.g., a Pentium^{™} type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the fluid processing device 10.

The controller 18 is configured and/or programmed to execute at least one fluid processing application but, more advantageously, is configured and/or programmed to execute a variety of different fluid processing applications. For example, the controller 18 may be configured and/or programmed to carry out one or more of the following: a double unit red blood cell collection procedure, a plasma collection procedure, a platelet-rich plasma/red blood cell collection procedure, a red blood cell/platelet/plasma collection procedure, a platelet collection procedure, a platelet/plasma collection procedure, and a mononuclear cell collection procedure. Additional or alternative procedure applications (e.g., plasma exchange, red blood cell exchange, and photopheresis) can be included without departing from the scope of the present disclosure.

More particularly, in carrying out any one of these fluid processing applications, the controller 18 is configured and/or programmed to control one or more of the following tasks: drawing fluid into a fluid flow circuit 12 mounted to the fluid processing device 10, conveying fluid through the fluid flow circuit 12 to a location for separation (i.e., into the spinning membrane separator 26 or the centrifuge chamber 32 of the fluid flow circuit 12), separating the fluid into two or more components as desired, and conveying the separated components into storage containers, to a second location for further separation (e.g., into whichever of the spinning membrane separator 26 and centrifuge chamber 32 that was not used in the initial separation stage), or to a recipient (which may be the source from which the fluid was originally drawn).

This may include instructing the spinning membrane separator drive unit 14 and/or the centrifugal separator 16 to operate at a particular rotational speed and instructing a pump to convey fluid through a portion of the fluid flow circuit 12 at a particular flow rate. Hence, while it may be described herein that a particular component of the fluid processing device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the fluid processing device 10 to monitor various aspects of the operation of the fluid processing device 10 and characteristics of the fluid and separated fluid components as they flow through the fluid flow circuit 12. If the operation of any of the components and/or one or more characteristics of the fluid or separated fluid components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 52 of the interface monitoring assembly and the centrifugal separator sensor M1. The signals that the controller 18 receives from the light detector 52 are indicative of the location of an interface between the separated fluid components within the centrifuge chamber 32, while the signals from the centrifugal separator sensor M1 indicate whether the target interface location should be adjusted. If the controller 18 determines that the interface is in the wrong location, then it can issue commands to the appropriate components of the fluid processing device 10 to modify their operation so as to move the interface to the proper location. For example, the controller 18 may instruct the pump system to cause fluid to flow into the centrifuge chamber 32 at a different rate and/or for a separated fluid component to be removed from the centrifuge chamber 32 at a different rate and/or for the centrifuge chamber 32 to be spun at a different speed by the centrifugal separator 16.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

### II. The Disposable Fluid Flow Circuit

### A. Overview

As for the fluid flow circuit or flow set 12 (Fig. 2), it is intended to be a sterile, single use, disposable item. Before beginning a given procedure, the operator loads various components of the fluid flow circuit 12 in the case 20 in association with the fluid processing device 10. The controller 18 implements the procedure based upon preset protocols, taking into account other input from the operator. Upon completing the procedure, the operator removes the fluid flow circuit 12 from association with the fluid processing device 10. The portions of the fluid flow circuit 12 holding the collected fluid component or components (e.g., collection containers or bags) are removed from the case 20 and retained for storage, immediate use, or further processing. The remainder of the fluid flow circuit 12 is removed from the case 20 and discarded.

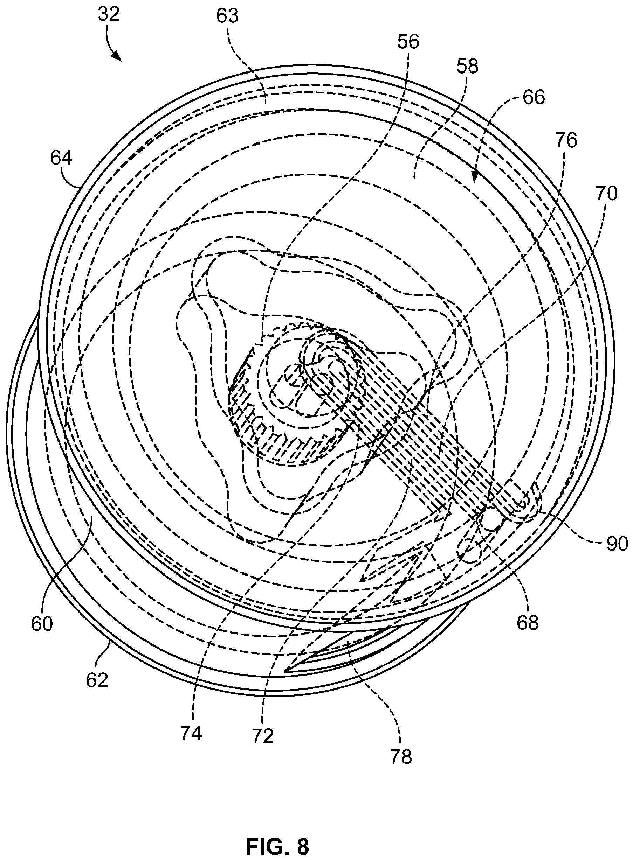

A variety of different disposable fluid flow circuits may be used in combination with the fluid processing device 10, with the appropriate fluid flow circuit depending on the procedure to be carried out using the system. Generally speaking, though, the fluid flow circuit 12 includes a cassette 48 (Fig. 4) to which the other components of the fluid flow circuit 12 are connected by flexible tubing or conduits. The other components may include a plurality of fluid containers F1-F7 (for holding fluid to be processed, a separated fluid component, a priming fluid, or an additive solution, for example), one or more fluid source access devices (e.g., a connector for accessing fluid within a fluid container), a centrifuge chamber 32 (Figs. 8-10), and (optionally) a spinning membrane separator 26.

Fig. 2 illustrates an exemplary fluid flow circuit 12 having a single fluid access device (e.g., a phlebotomy needle) for alternately drawing fluid into the fluid flow circuit 12 and conveying fluid out of the fluid flow circuit 12. It should be understood that the illustrated fluid flow circuit 12 is merely exemplary and that the principles described herein may be employed with differently configured fluid flow circuits. This may include fluid flow circuits having a pair of fluid access devices, with one dedicated to drawing fluid into the fluid flow circuit and other being dedicated to conveying fluid out of the fluid flow circuit.

### B. Cassette And Tubing

The cassette 48 (Fig. 4) provides a centralized, programmable, integrated platform for all the pumping and many of the valving functions required for a given fluid processing procedure. In one embodiment, the cassette 48 is similarly configured to the cassette of U.S. Patent No. 5,868,696, but is adapted to include additional components (e.g., more tubing loops T1-T6) and functionality.

In use, the cassette 48 is mounted to the cassette station 54 of the fluid processing device 10, with a flexible diaphragm of the cassette 48 placed into contact with the cassette station 54. The flexible diaphragm overlays an array of interior cavities formed by the body of the cassette 48. The different interior cavities define sensor stations S1-S4, valve stations C1-C9, and a plurality of flow paths or conduits L1-L23 (Fig. 2). The side of the cassette 48 opposite the flexible diaphragm may be sealed by another flexible diaphragm or a rigid cover, thereby sealing fluid flow through the cassette 48 from the outside environment.

Each sensor station S1-S4 is aligned with an associated pressure sensor A1-A4 of the cassette station 54, with each pressure sensor A1-A4 capable of monitoring the pressure within the associated sensor station S1-S4. Each valve station C1-C9 is aligned with an associated valve V1-V9, and may define one or more ports that allow fluid communication between the valve station C1-C9 and another interior cavity of the cassette 48 (e.g., a flow path). As described above, each valve V1-V9 is movable under command of the controller 18 to move between a plurality of positions (e.g., between a retracted or lowered position and an actuated or raised position) to selectively contact the valve stations C1-C9 of the cassette 48. In the actuated position, a valve V1-V9 engages the associated valve station C1-C9 to close one or more of its ports to prevent fluid flow therethrough. In the retracted position, a valve V1-V9 is disengaged from the associated valve station C1-C9 (or less forcefully contacts the associated valve station C1-C9 than when in the actuated position) to open one or more ports associated with the valve station C1-C9, thereby allowing fluid flow therethrough.

As described, a plurality of tubing loops T1-T6 extend from the side surface of the cassette 48 to interact with pumps P1-P6 of the fluid processing device 10. In the illustrated embodiment, six tubing loops T1-T6 extend from the cassette 48 to be received by a different one of six pumps P1-P6, but in other embodiments, a procedure may not require use of all of the pumps P1-P6, in which case the cassette 48 may include fewer than six tubing loops. The different pumps P1-P6 may interact with the tubing loops T1-T6 of the cassette 48 to perform different tasks during a separation procedure. Certain procedures require fewer than all of the sensor stations, valve stations, and/or tubing loops illustrated in the exemplary cassette 48 of Fig. 4, such that it should be understood that the cassettes of different fluid flow circuits 12 may be differently configured (e.g., with fewer sensor stations, valve stations, and/or tubing loops) without departing from the scope of the present disclosure.

Additional tubing or conduits extend from the side surface of the cassette 48 to connect to the other components of the fluid flow circuit 12, such as the various fluid containers F1-F7, the spinning membrane separator 26, and the centrifuge chamber 32. The number and content of the various fluid containers F1-F7 depends upon the procedure for which the fluid flow circuit 12 is used. The tubing connected to the centrifuge chamber 32 (which includes one inlet conduit and two outlet conduits) may be aggregated into an umbilicus 46 (Fig. 3) that is engaged by the yoke member 44 of the centrifugal separator 16 (as described above) to cause the umbilicus 46 to orbit around and spin or rotate the centrifuge chamber 32 during a separation procedure.

Various additional components may be incorporated into the tubing leading out of the cassette 48 or into one of the cavities of the cassette 48. For example, as shown in Fig. 2, a manual clamp 51 may be associated with a line or lines leading to the fluid source and/or fluid recipient, a return line filter 53 (e.g., a microaggregate filter) may be associated with a line leading to a fluid recipient, filters may be positioned upstream of one or more of the fluid containers to remove a substance (e.g., leukocytes) from a separated component (e.g., red blood cells) flowing into the fluid container, and/or an air trap 55 may be positioned on a line upstream of the centrifuge chamber 32.

### C. Centrifuge Chamber

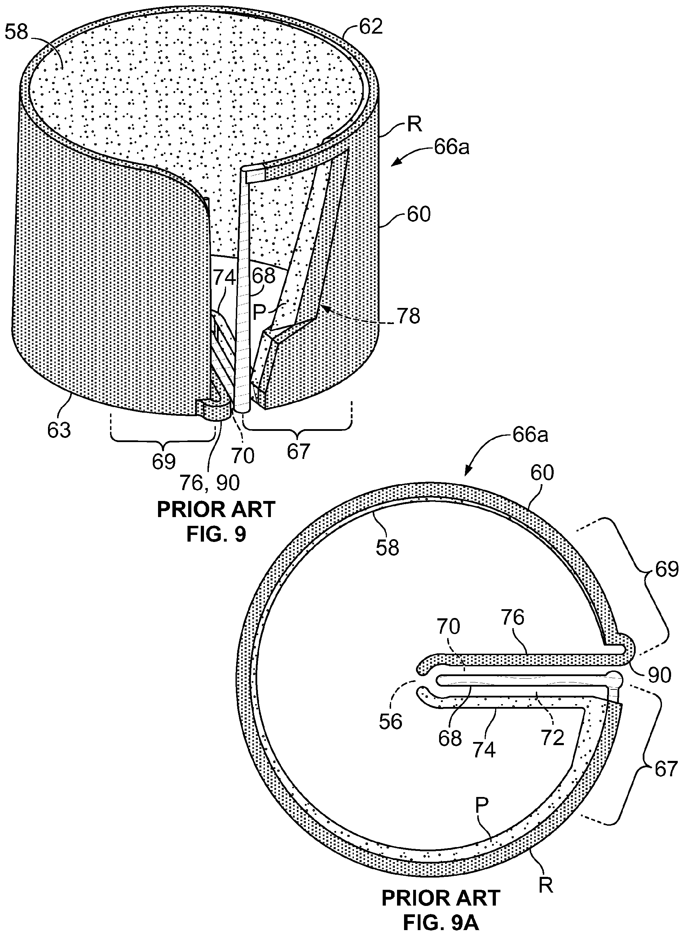

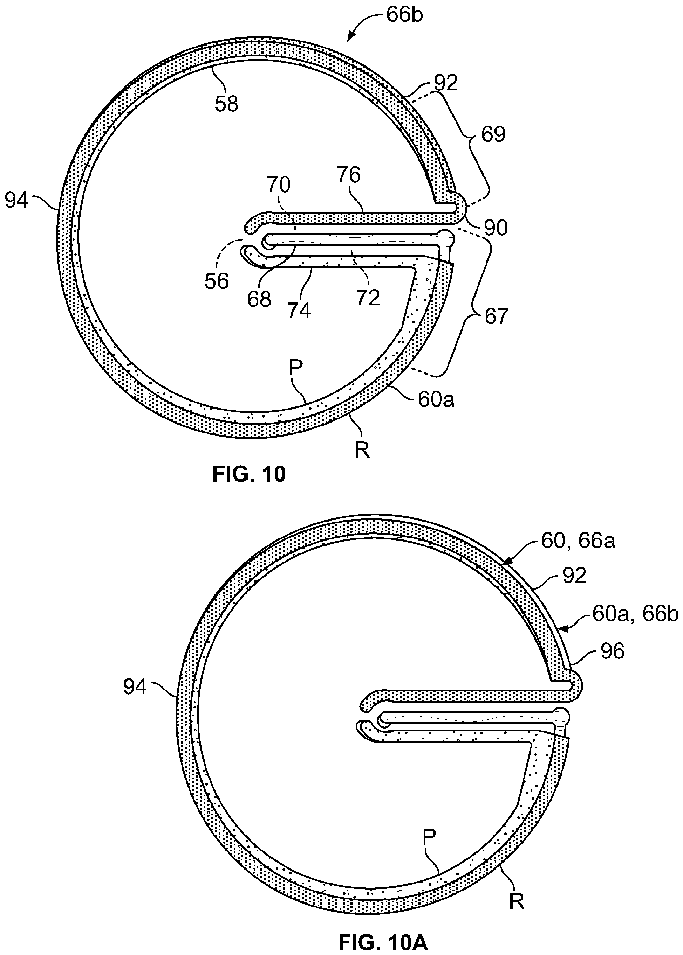

An exemplary centrifuge chamber 32 is shown in Fig. 8. Figs. 9 and 9A illustrate a conventionally configured fluid flow path or separation channel that may be defined by the centrifuge chamber 32. Fig. 10 illustrates an exemplary fluid flow path or separation channel according to an aspect of the present disclosure that may be defined by the centrifuge chamber 32, with Fig. 10A comparing the channel of Fig. 10 to a conventional channel. The differences between the separation channel of Fig. 10 and the conventional channel will be described in greater detail herein.

In the illustrated embodiment, the body of the centrifuge chamber 32 is pre-formed in a desired shape and configuration (e.g., by injection molding) from a rigid, biocompatible plastic material, such as a non-plasticized medical grade acrylonitrile-butadiene-styrene (ABS). All contours, ports, channels, and walls that affect the fluid separation process are preformed in a single, injection molded operation. Alternatively, the centrifuge chamber 32 can be formed by separate molded parts, either by nesting cup-shaped subassemblies or two symmetric halves.

A central hub 56 of the centrifuge chamber 32 (Fig. 8) coincides with the rotational axis 38 when the chamber 32 is mounted within the centrifuge compartment 34. The central hub 56 includes a shaped receptacle that is suitable for receiving an end of the umbilicus 46 of the fluid flow circuit 12 (Fig. 3). A suitable receptacle and the manner in which the umbilicus 46 may cooperate with the receptacle to deliver fluid to and remove fluid from the centrifuge chamber 32 are described in greater detail in U.S. Patent No. 8,075,468.

The illustrated centrifuge chamber 32 has radially spaced apart inner (low-G) and outer (high-G) walls 58 and 60 extending about the central hub 56. The low-G and high-G walls 58 and 60 extend from a bottom end wall 62 and an open top end 63. It should be understood that the terms "top" and "bottom" are not intended to restrict the structure or orientation of the centrifuge chamber 32 (e.g., Fig. 9 shows the bottom end 62 positioned above the top end 63), but rather are used to describe the various aspects of the centrifuge chamber 32. A cover 64 is associated with the open top end 63 of the centrifuge chamber 32, with the cover 64 comprising a simple flat part that can be easily welded or otherwise secured to the body of the centrifuge chamber 32. Because all features that affect the separation process are incorporated into one injection molded component, any tolerance differences between the cover 64 and the body of the centrifuge chamber 32 will not affect the separation efficiencies of the centrifuge chamber 32. The low-G and high-G walls 58 and 60, the bottom 62, and the cover 64 together define an enclosed, generally annular channel 66. As noted above, exemplary separation channels are illustrated in Figs. 9-10A and will be described in greater detail herein.

In order to enable injection molding of the centrifuge chamber 32, a 1 ° inward taper (toward the central axis of the chamber 32, from the open top end 63 to the bottom end 62) may be incorporated into the high-G wall 60, while a 1° outward taper (away from the central axis of the chamber 32, from the top end 63 to the bottom end 62) may be incorporated into the low-G wall 58. Thus, the width of the separation channel 66 (i.e., the distance between the low-G and high-G walls 58 and 60) will tend to vary along the height of the separation channel 66. As will be described in greater detail, the width of the separation channel 66 about the axis (i.e., from an upstream end 67 of the channel 66 to a downstream end 69) may vary, so it should be understood that any reference to the width of a separation channel 66 about the axis is with regard to the width of the channel 66 at a particular axial position or height.

A plurality of radial walls extend from the central hub 56 to the separation channel 66 with an inlet passage 68 being defined between two of the radial walls 70 and 72 which, in one flow configuration, allows fluid to flow from the umbilicus 46 into the channel 66. One of the radial walls 70 (which may be referred to as a "terminal wall") joins the high-G wall 60 and separates the upstream end 67 of the channel 66 from a downstream end 69. As used herein, the terms "upstream end" and "downstream end," when used in regard to regions of the separation channel 66, may refer to the first quarter or quadrant of the channel 66 (i.e., the region encompassing approximately 90° of the channel 66 on the side of the terminal wall 70 in which fluid enters the channel 66) and the last quarter or quadrant of the channel 66 (i.e., the region encompassing approximately 90° of the channel 66 on the side of the terminal wall 70 opposite the upstream end 67 of the channel 66), respectively. These terms are most frequently used herein to refer to the position of various components or formations associated with the separation channel 66 (e.g., the inlet passage 68 opens into the channel 66 at the upstream end 67 of the channel 66) such that, in certain embodiments (depending on the configurations of the components described as being present at the upstream end 67 or downstream end 69 of the separation channel 66), the terms may refer to smaller regions of the separation channel 66, which may include (for example) the term "upstream end" referring to only the first 45° or 30° or less of the channel 66 and the term "downstream end" referring to only the last 45° or 30° or less of the channel 66.

The illustrated centrifuge chamber 32 further includes first and second outlet passages 74 and 76, respectively, which may be defined by opposing surfaces of interior radial walls. Both the first and second outlet passages 74 and 76 extend between the central hub 56 and the separation channel 66. The first (low-G) outlet passage 74 extends radially inward from an opening which, in the illustrated embodiment, is located at the low-G wall 58, while the second (high-G) outlet passage 76 extends radially inward from an opening that is associated with the high-G wall 60. The illustrated low-G outlet passage 74 is positioned adjacent to the inlet passage 68 (at the upstream end 67 of the separation channel 66), while the high-G outlet passage 76 may be positioned at the opposite, downstream end 69 of the channel 66.

Additional features of exemplary separation channels will be described in greater detail herein.

### III. Centrifugal Separation And Interface Detection Principles

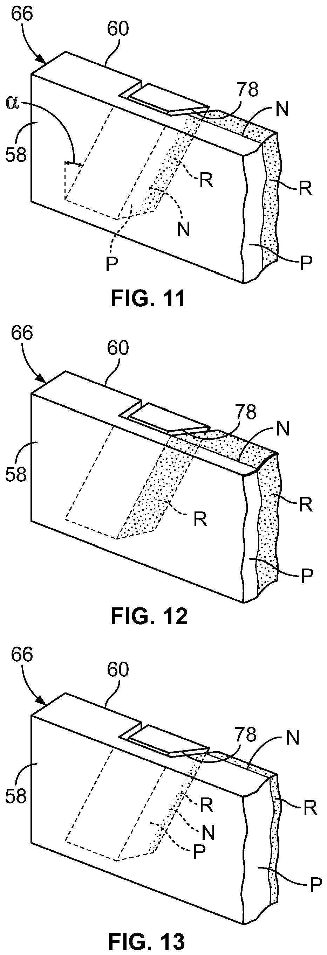

Fluid flowed into the separation channel 66 separates into an optically dense layer "R" and a less optically dense layer "P" (Figs. 11-13) as the centrifuge chamber 32 is rotated about the rotational axis 38. The optically dense layer R forms as larger and/or heavier fluid particles move under the influence of centrifugal force toward the high-G wall 60. If the fluid being separated is blood, the optically dense layer R will typically include red blood cells but, depending on the speed at which the centrifuge chamber 32 is rotated, other cellular components (e.g., larger white blood cells) may also be present in the optically dense layer R.

If the fluid being separated is blood, the less optically dense layer P typically includes a plasma constituent, such as platelet-rich plasma. Depending on the speed at which the centrifuge chamber 32 is rotated and the length of time that the blood is resident therein, other components (e.g., smaller white blood cells) may also be present in the less optically dense layer P.

In one embodiment, fluid introduced into the separation channel 66 via the inlet passage 68 will travel in a generally clockwise direction (in the orientation of Fig. 8) as the optically dense layer R separates from the less optically dense layer P. The optically dense layer R continues moving in the clockwise direction as it travels the length of the separation channel 66 along the high-G wall 60, from the upstream end 67 to the downstream end 69, where it exits the channel 66 via the high-G outlet passage 76. The less optically dense layer P separated from the optically dense layer R reverses direction, moving counterclockwise along the low-G wall 58 to the low-G outlet passage 74, adjacent to the inlet passage 68.

The transition between the optically dense layer R and the less optically dense layer P may be referred to as the interface "N". If the fluid being separated is blood, a buffy coat containing mononuclear cells and peripheral blood stem cells may be located at the interface N. The location of the interface N within the separation channel 66 of the centrifuge chamber 32 can dynamically shift during fluid processing, as Figs. 11-13 show. If the location of the interface N is too high (that is, if it is too close to the low-G wall 58 and the low-G outlet passage 74, as in Fig. 12), red blood cells can flow into the low-G outlet passage 74, potentially adversely affecting the quality of the low density components (platelet-rich plasma). On the other hand, if the location of the interface N is too low (that is, if it resides too far away from the low-G wall 58, as Fig. 13 shows), the collection efficiency of the system may be impaired. The ideal or target interface location may be experimentally determined, which may vary depending on any of a number of factors (e.g., the configuration of the centrifuge chamber 32, the rate at which the centrifuge chamber 32 is rotated about the rotational axis 38, etc.).

As described above, the fluid processing device 10 may include an interface monitoring assembly (including the light source 50 and the light detector 52), a centrifugal separator sensor M1, and a controller 18 with an interface control module to monitor and, as necessary, adjust or correct the position of the interface N. In the illustrated embodiment, the centrifuge chamber 32 is formed with a ramp 78 extending from the high-G wall 60 at an angle α across at least a portion of the separation channel 66 (Figs. 8 and 11-13). The angle α, measured with respect to the rotational axis 38 is about 25° in one embodiment. Figs. 11-13 show the orientation of the ramp 78 when viewed from the low-G wall 58 of the centrifuge chamber 32. Although it describes a flexible separation chamber, the general structure and function of the ramp 78 may be better understood with reference to U.S. Patent No. 5,632,893, which is hereby incorporated herein by reference. The ramp 78 may be positioned at any of a number of locations between the upstream and downstream ends 67 and 69 of the separation channel 66, but in one embodiment, the ramp 78 may be positioned generally adjacent to the low-G outlet passage 74, in the path of fluid and/or a fluid component moving from the inlet passage 68 to the low-G outlet passage 74.

The ramp 78 makes the interface N between the optically dense layer R and the less optically dense layer P more discernible for detection, displaying the optically dense layer R, less optically dense layer P, and interface N for viewing through a light-transmissive portion of the centrifuge chamber 32. To that end, the ramp 78 and at least the portion of the centrifuge chamber 32 angularly aligned with the ramp 78 may be formed of a light-transmissive material, although it may be advantageous for the entire centrifuge chamber 32 to be formed of the same light-transmissive material.

In the illustrated embodiment, the light source 50 of the interface monitoring system is associated with a fixture or wall of the centrifuge compartment 34 and oriented to emit a light L that is directed toward the rotational axis 38 of the centrifugal separator 16, as shown in Figs. 5-7. If the light detector 52 is positioned at an angle with respect to the light source 50 (as in the illustrated embodiment), the light L emitted by the light source 50 must be redirected from its initial path before it will reach the light detector 52. In the illustrated embodiment, the light L is redirected by a reflector that is associated with a light-transmissive portion of the inner side wall portion 58, as shown in Figs. 5 and 6. The reflector may be a separate piece that is secured to the low-G wall 58 (e.g., by being bonded thereto) or may be integrally formed with the body of the centrifuge chamber 32.

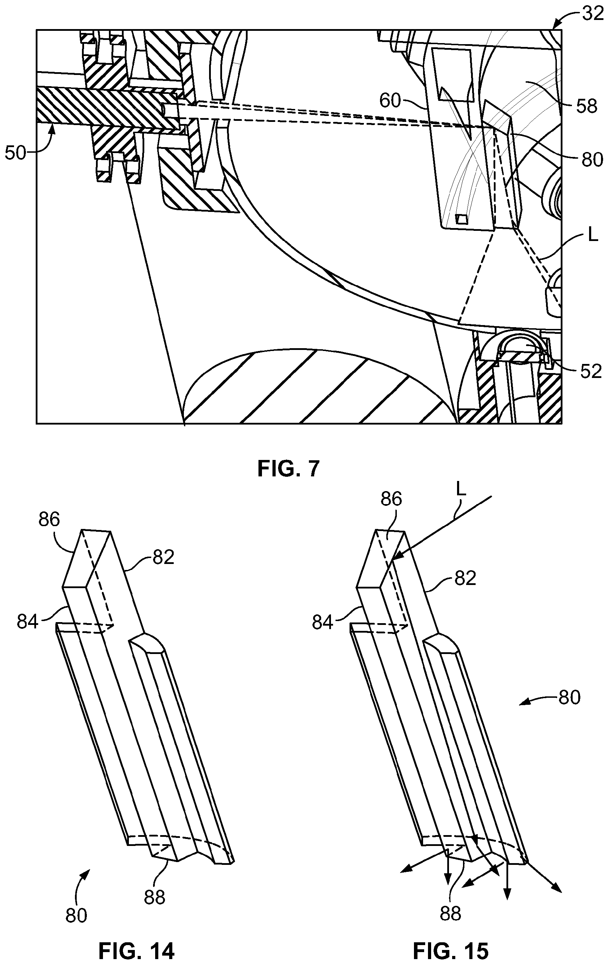

In one embodiment, the reflector may be a reflective surface, such as a mirror, that is oriented (e.g., at a 45° angle) to direct light L emitted by the light source 50 to the light detector 52. In another embodiment, the reflector is provided as a prismatic reflector 80 (Figs. 7, 14, and 15), which is formed of a light-transmissive material (e.g., a clear plastic material) and has inner and outer walls 82 and 84 and first and second end walls 86 and 88 (Fig. 14). The inner wall 82 is positioned against the low-G wall 58 of the centrifuge chamber 32 and is oriented substantially perpendicular to the initial path of the light L from the light source 50. This allows light L from the light source 50 to enter into the prismatic reflector 80 via the inner wall 82 while continuing along its initial path. The light L continues through the prismatic reflector 80 along its initial path until it encounters the first end wall 86. The first end wall 86 is oriented at an angle (e.g., an approximately 45° angle) with respect to the inner wall 82 and the second end wall 88, causing the light L to be redirected within the prismatic reflector 80, rather than exiting the prismatic reflector 80 via the first end wall 86.

The first end wall 86 directs the light L at an angle to its initial path (which may be an approximately 90° angle, directing it from a path toward the rotational axis 38 to a path that is generally parallel to the rotational axis 38) toward the second end wall 88 (Fig. 15). The first end wall 86 and the inner and outer walls 82 and 84 of the prismatic reflector 80 may be configured to transmit the redirected light L from the first end wall 86 to the second end wall 88 by total internal reflection. The second end wall 88 is oriented substantially perpendicular to the redirected path of the light L through the prismatic reflector 80, such that the light L will exit the prismatic reflector 80 via the second end wall 88, continuing along its redirected path. In one embodiment, the second end wall 88 is roughened or textured or otherwise treated or conditioned to diffuse the light L as it exits the prismatic reflector 80, which may better ensure that the light L reaches the light detector 52 (Fig. 7).

The prismatic reflector 80 may be angularly aligned with the ramp 78, such that the light L from the light source 50 will only enter into the prismatic reflector 80 when the ramp 78 has been rotated into the path of the light L. At all other times (when the ramp 78 is not in the path of the light L), the light L will not reach the prismatic reflector 80 and, thus, will not reach the light detector 52.

Upon the ramp 78 first being rotated into the path of the light L from the light source 50, the light L will begin to reach the prismatic reflector 80, which directs the light L to the light detector 52. This causes the voltage output of the light detector 52 (i.e., the signal transmitted from the light detector 52 to the controller 18) to increase to a non-zero value or state. The ramp 78 and prismatic reflector 80 are eventually rotated out of alignment with the light source 50, at which time no light L will reach the prismatic reflector 80 and the voltage output of the light detector 52 will return to a low- or zero-state.

During the time that the ramp 78 and prismatic reflector 80 are rotated through the path of the light L from the light source 50, the light L continues through the separation channel 66 and the fluids in the channel 66. At least a portion of the light L (i.e., the portion not absorbed or reflected by the fluids) exits the separation channel 66 by striking and entering a light-transmissive portion of the low-G wall 58. The light L passes through the low-G wall 58 and enters the prismatic reflector 80, which redirects the light L from its initial path to the light detector 52, as described above.

The light detector 52 generates a signal that is transmitted to the interface control module of the controller 18, which can determine the location of the interface N on the ramp 78. In one embodiment, the location of the interface N is associated with a change in the amount of light L that is transmitted through the less optically dense layer P and the optically dense layer R. For example, the light source 50 may be configured to emit a light L that is more readily transmitted by platelet-rich plasma than by red blood cells, such as red visible light (from a laser or a differently configured light source L), which is substantially absorbed by red blood cells. The less optically dense layer P and the optically dense layer R each occupy a certain portion of the ramp 78, with the light detector 52 receiving different amounts of light L depending on whether the light L travels through the less optically dense layer P on the ramp 78 or the optically dense layer R on the ramp 78. The percentage of the ramp 78 occupied by each layer is related to the location of the interface N in the channel 66. Thus, by measuring the amount of time that the voltage output or signal from the light detector 52 is relatively high (corresponding to the time during which the light L is passing through only the less optically dense layer P on the ramp 78), the controller 18 may determine the location of the interface N and take steps to correct the location of the interface N, if necessary. An exemplary approach to adjustment of the position of the interface N is described in greater detail in PCT Patent Application Publication No. WO 2018/053217 A1.

### IV. Separation Channel Configuration

Turning back now to the configuration of the separation channel 66 defined by the centrifuge chamber 32, Figs. 9 and 9A illustrate a conventional separation channel 66a that may be defined by the centrifuge chamber 32. It should be understood that, for illustrative purposes, the orientation of the separation channel 66a shown in Fig. 9 is reversed with respect to the orientation of the centrifuge chamber 32 shown in Fig. 8 (i.e., the bottom end 62 is pictured at the top of Fig. 9).

While the various surfaces and formations of the centrifuge chamber are not visible in Figs. 9-10A, it should be clear that the illustrated separation channels are entirely defined by the corresponding chamber structures and surfaces described herein and shown in Fig. 8. Thus, for the sake of simplicity and brevity, when describing the separation channels of Figs. 9-10A, reference may simply be made to the centrifuge chamber itself and the various surfaces and formations of the chamber (e.g., stating that the chambers of Figs. 9-10A have a particularly configured ramp, rather than stating that the chamber defining the illustrated channel has a particularly configured ramp). Similarly, Figs. 9-10A may be annotated with reference numerals (with broken lead lines) representing the chamber surface defining a particular feature of the illustrated separation channel, rather than employing a new reference numeral for the channel feature arising from the chamber surface (e.g., identifying a channel feature defined by a ramp of a chamber as the ramp instead of employing a unique identifier for that region of the channel itself).

As described above, the inlet passage 68 and low-G outlet passage 74 open into the separation channel 66a at the upstream end 67 of the channel 66a, with the ramp 78 extending across a portion of the upstream end 67 of the channel 66a. The high-G outlet passage 76 opens into the separation channel 66a at the downstream end 69 of the channel 66a. All of the radial walls extending between the central hub 56 and the separation channel 66a to define the inlet passage 68 and the outlet passages 74 and 76 are defined at the open top end 63 of the centrifuge chamber 32 (which is shown at the bottom of Fig. 9), though the inlet passage 68 is configured so as to open into the channel 66a at the bottom end 62, while the outlet passages 74 and 76 open into the channel 66a at the top end 63. This configuration may be advantageous to increase the distance that the separated fluid components must travel through the separation channel 66a before exiting via either outlet passage 74, 76, which may improve the purity of the separated fluid components.

The low-G wall 58 has a radius that is greater at the downstream end 69 of the separation channel 66a than at the upstream end 67 (at each axial position, as explained above). Fig. 9A shows the radius of the low-G wall 58 gradually increasing from the upstream end 67 of the separation channel 66a (just downstream of the ramp 78) to the downstream end 69 of the channel 66a, which may include the low-G wall 58 being configured as a uniform spiral downstream of the ramp 78. This configuration of the low-G wall 58 may be advantageous when separating blood into packed red blood cells (exiting the separation channel 66a via the high-G outlet passage 76) and platelet-rich plasma (exiting the channel 66a via the low-G outlet passage 74) as it causes plasma that continues to release from the red blood cells downstream of the inlet passage 68 to flow in the direction of increasing plasma thickness (i.e., back upstream toward and into the low-G outlet passage 74). The backflow of plasma helps to pull platelets that were not captured by the initial elutriation separation process towards the upstream end 67 of the separation channel 66a, where they can be captured and pulled out of the channel 66a by the high-velocity plasma stream present near the low-G outlet passage 74.

As for the high-G wall 60, it has a uniform radius from the upstream end 67 of the separation channel 66a to the downstream end 69 (at each axial position). The separation channel 66a is shown with a passage 90 recessed into the high-G wall 60 at the downstream end 69 of the channel 66a, which may be advantageous in order to prevent any of the separated fluid component at the low-G wall 58 (e.g., platelet-rich plasma) from entering the high-G outlet passage 76.

On account of the radius of the low-G wall 58 increasing from the upstream end 67 of the separation channel 66a to the downstream end 69, while the radius of the high-G wall 60 remains uniform, the width of the channel 66a (at each axial position) decreases from the upstream end 67 to the downstream end 69, as best shown in Fig. 9A. The downstream end 69 of the separation channel 66a may be thin enough to cause difficulties in manufacturing the centrifuge chamber 32, such as when the necessary mold tooling core is so thin as to be susceptible to premature failure from the repeated high stresses during the molding process. One approach to decreasing the risk of failure of the mold tooling core would be to increase the uniform radius of the high-G wall 60 to widen the separation channel 66a at all locations, which would allow for a thicker, stronger core. However, such an approach may be disadvantageous to the extent that it would significantly increase the volume of the separation channel 66a (e.g., from 51.4 mL to 73.2 mL), which may be problematic when platelets are to be collected from blood (on account of extracorporeal volume being preferably minimized or at least reduced for platelet collection procedures).

Accordingly, it has been found that, rather than uniformly increasing the radius of the high-G wall 60, it is preferable to instead increase the radius of only a portion of the high-G wall 60 or to increase the radius of the high-G wall 60 to different degrees at different positions along the length of the separation channel 66. Fig. 10 illustrates one possible configuration of a separation channel 66b defined by a high-G wall 60a having a non-uniform radius, according to an aspect of the present disclosure. Fig. 10A illustrates the different configurations of the separation channels 66a and 66b and high-G walls 60 and 60a.

As described above, the conventional separation channel 66a is thinnest at its downstream end 69. Thus, in order to increase the width of the separation channel at its downstream end 69 (presuming no changes are made to the configuration of the low-G wall 58) and allow for a thicker mold tooling core, the high-G wall 60a of Figs. 10 and 10A has a greater radius at the downstream end 69 of the separation channel 60b than at the upstream end 67 (at each axial position). Figs. 10 and 10A illustrate a configuration in which the high-G wall 60a includes a tapered section 92 extending along a portion of the length of the separation channel 66b (which portion includes the downstream end 69 of the channel 66b). The radius of the high-G wall 60a gradually increases (at each axial position) from an upstream portion 94 of the tapered section 92 to a downstream portion 96 of the tapered section 92 (which coincides with the downstream end 69 of the separation channel 66b). This may include the tapered section 92 being configured as a uniform spiral.

In an exemplary embodiment, the tapered section 92 is configured as a uniform spiral having a pitch of 0.307 cm, which increases the radius of the high-G wall 60a at the downstream end 69 of the separation channel 66b by approximately 1.5 mm (compared to the radius of the conventional high-G wall 60 at the downstream end 69 of the channel 66a). However, the position at which the tapered section 92 begins, the rate at which the radius of the high-G wall 60b increases along the tapered section 92, and the degree to which the radius of the high-G wall 60a at the downstream end 69 of the separation channel 66b is greater than the radius of the high-G wall 60a at the upstream portion 94 of the tapered section 92 may vary without departing from the scope of the present disclosure. For example, in the illustrated embodiment, the tapered section 92 extends along an approximately semicircular portion of the separation channel 66b (i.e., along the second half of the channel 66b), with the high-G wall 60a having a uniform radius (at each axial position) upstream of the tapered section 92. If the low-G wall 58 is provided according to conventional design (with a radius that gradually increases from the upstream end 67 of the separation channel 66b to the downstream end 69), the width of the channel 66b will decrease (at each axial position) from the upstream end 67 of the channel 66b to the end of the portion of the high-G wall 60a having a uniform radius. As for the portion of the separation channel 66b extending along the tapered section 92 of the high-G wall 60a, it may have a substantially uniform width (if the curvature of the tapered section 92 matches the curvature of the low-G wall 58) or a non-uniform width (if the curvature of the tapered section 92 differs from the curvature of the low-G wall 58) at each axial position.

According to an alternative embodiment, the tapered section 92 of the high-G wall 60a may extend from the upstream end 67 of the separation channel 66b to the downstream end 69, in which case the radius of the high-G wall 60a gradually increases from the upstream end 67 of the channel 66b to the downstream end 69 (just upstream of the passage 90 recessed into the high-G wall 60a at the high-G outlet passage 76). This may include the tapered section 92 (i.e., the entire high-G wall 60a) being configured as a uniform spiral upstream of the passage 90 recessed into the high-G wall 60a at the downstream end 69 of the separation channel 66b. In such an embodiment, the separation channel 66b may have a substantially uniform width (at each axial position) from the upstream end 67 of the channel 66b (just downstream of the ramp 78) to the downstream end 69 (just upstream of the passage 90 recessed into the high-G wall 60a) at each axial position, if the curvature of the high-G wall 60a matches the curvature of the low-G wall 58. On the other hand, if the curvature of the high-G wall 60a differs from the curvature of the low-G wall 58, the separation channel 66b will instead have a non-uniform width (at each axial position) from the upstream end 67 of the channel 66b to the downstream end 69.

### Aspects

Aspect 1. A fluid separation chamber for rotation about an axis, comprising: a central hub coinciding with the axis; a generally annular low-G wall and a generally annular high-G wall extending about the central hub in a spaced apart relationship to define therebetween a separation channel having an upstream end and a downstream end; and a plurality of radial walls extending from the central hub to the separation channel to define a terminal wall separating the upstream end of the separation channel from the downstream end of the separation channel, an inlet passage at the upstream end of the separation channel, and low-G and high-G outlet passages, wherein the high-G wall has a greater radius at the downstream end of the separation channel than at the upstream end of the separation channel at each axial position.

Aspect 2. The fluid separation chamber of Aspect 1, wherein the high-G wall includes a tapered section with a radius that gradually increases from an upstream portion of the tapered section to a downstream portion of the tapered section at each axial position.

Aspect 3. The fluid separation chamber of Aspect 2, wherein the tapered section of the high-G wall has a radius configured as a uniform spiral.

Aspect 4. The fluid separation chamber of any one of Aspects 2-3, wherein the high-G wall has a uniform radius upstream of the tapered section at each axial position.

Aspect 5. The fluid separation chamber of Aspect 4, wherein the tapered section extends along an approximately semicircular portion of the separation channel.

Aspect 6. The fluid separation chamber of any one of Aspects 2-3, wherein the tapered section extends from the upstream end of the separation channel to the downstream end of the separation channel.

Aspect 7. The fluid separation chamber of any one of the preceding Aspects, wherein the low-G wall has a greater radius at the downstream end of the separation channel than at the upstream end of the separation channel at each axial position.

Aspect 8. The fluid separation chamber of any one of the preceding Aspects, wherein the low-G wall has a radius that gradually increases from the upstream end of the separation channel to the downstream end of the separation channel at each axial position.

Aspect 9. The fluid separation chamber of any one of the preceding Aspects, wherein the low-G wall has a radius configured as a uniform spiral from the upstream end of the separation channel to the downstream end of the separation channel.

Aspect 10. The fluid separation chamber of Aspect 1, wherein the high-G wall includes a tapered section with a radius that gradually increases from an upstream portion of the tapered section to a downstream portion of the tapered section at each axial position, the high-G wall has a uniform radius upstream of the tapered section at each axial position, the low-G wall has a radius that gradually increases from the upstream end of the separation channel to the downstream end of the separation channel at each axial position, and the separation channel has a non-uniform width upstream of the tapered section at each axial position.

Aspect 11. The fluid separation chamber of Aspect 10, wherein the separation channel has a substantially uniform width along the tapered section at each axial position.

Aspect 12. The fluid separation chamber of Aspect 10, wherein the separation channel has a non-uniform width along the tapered section at each axial position.

Aspect 13. The fluid separation chamber of any one of Aspects 10-12, wherein the tapered section and the low-G wall each have a radius configured as a uniform spiral.

Aspect 14. The fluid separation chamber of Aspect 1, wherein each of the low-G and high-G walls has a radius configured as a uniform spiral from the upstream end of the separation channel to the downstream end of the separation channel.

Aspect 15. The fluid separation chamber of Aspect 14, wherein the separation channel has a substantially uniform width from the upstream end of the separation channel to the downstream end of the separation channel at each axial position.

Aspect 16. The fluid separation chamber of Aspect 14, wherein the separation channel has a non-uniform width from the upstream end of the separation channel to the downstream end of the separation channel at each axial position.

Aspect 17. The fluid separation chamber of any one of the preceding Aspects, wherein the low-G outlet passage opens into the separation channel at the upstream end of the separation channel.

Aspect 18. The fluid separation chamber of any one of the preceding Aspects, wherein the high-G outlet passage opens into the separation channel at the downstream end of the separation channel.

Aspect 19. The fluid separation chamber of any one of the preceding Aspects, wherein the low-G and high-G outlet passages open into the separation channel at a top end of the separation channel.

Aspect 20. The fluid separation chamber of any one of the preceding Aspects, wherein the inlet passage opens into the separation channel at a bottom end of the separation channel.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A fluid separation chamber (32) for rotation about an axis (38), comprising:
a central hub (56) coinciding with the axis (38);
a generally annular low-G wall (58) and a generally annular high-G wall (60a) extending about the central hub (56) in a spaced apart relationship to define therebetween a separation channel (66b) having an upstream end (67) and a downstream end (69); and
a plurality of radial walls (70, 72) extending from the central hub (56) to the separation channel (66b) to define a terminal wall separating the upstream end (67) of the separation channel (66b) from the downstream end (69) of the separation channel (66b), an inlet passage (68) at the upstream end (67) of the separation channel (66b), and low-G and high-G outlet passages (74, 76), wherein the high-G wall (60a) has a greater radius at the downstream end (69) of the separation channel (66b) than at the upstream end (67) of the separation channel (66b) at each axial position.

2. The fluid separation chamber (32) of claim 1, wherein the high-G wall (60a) includes a tapered section (92) with a radius that gradually increases from an upstream portion (94) of the tapered section (92) to a downstream portion (96) of the tapered section (92) at each axial position, with the tapered section (92) of the high-G wall (60a) preferably having a radius configured as a uniform spiral.

3. The fluid separation chamber (32) of claim 2, wherein the high-G wall (60a) has a uniform radius upstream of the tapered section (92) at each axial position, with the tapered section (92) preferably extending along an approximately semicircular portion of the separation channel (66b).

4. The fluid separation chamber (32) of claim 2, wherein the tapered section (92) extends from the upstream end (67) of the separation channel (66b) to the downstream end (69) of the separation channel (66b).

5. The fluid separation chamber (32) of any one of the preceding claims, wherein the low-G wall (58) has a greater radius at the downstream end (69) of the separation channel (66b) than at the upstream end (67) of the separation channel (66b) at each axial position, with the low-G wall (58) preferably having a radius configured as a uniform spiral that gradually increases from the upstream end (67) of the separation channel (66b) to the downstream end (69) of the separation channel (66b) at each axial position.

6. The fluid separation chamber (32) of claim 1, wherein
the high-G wall (60a) includes a tapered section (92) with a radius that gradually increases from an upstream portion (94) of the tapered section (92) to a downstream portion (96) of the tapered section (92) at each axial position,
the high-G wall (60a) has a uniform radius upstream of the tapered section (92) at each axial position,
the low-G wall (58) has a radius that gradually increases from the upstream end (67) of the separation channel (66b) to the downstream end (69) of the separation channel (66b) at each axial position, and
the separation channel (66b) has a non-uniform width upstream of the tapered section (92) at each axial position.

7. The fluid separation chamber (32) of claim 6, wherein the separation channel (66b) has a substantially uniform width along the tapered section (92) at each axial position.

8. The fluid separation chamber (32) of claim 6, wherein the separation channel (66b) has a non-uniform width along the tapered section (92) at each axial position.

9. The fluid separation chamber (32) of any one of claims 6-8, wherein the tapered section (92) and the low-G wall (58) each have a radius configured as a uniform spiral.

10. The fluid separation chamber (32) of claim 1, wherein each of the low-G and high-G walls (58, 60a) has a radius configured as a uniform spiral from the upstream end (67) of the separation channel (66b) to the downstream end (69) of the separation channel (66b).

11. The fluid separation chamber (32) of claim 10, wherein the separation channel (66b) has a substantially uniform width from the upstream end (67) of the separation channel (66b) to the downstream end (69) of the separation channel (66b) at each axial position.

12. The fluid separation chamber (32) of claim 10, wherein the separation channel (66b) has a non-uniform width from the upstream end (67) of the separation channel (66b) to the downstream end (69) of the separation channel (66b) at each axial position.

13. The fluid separation chamber (32) of any one of the preceding claims, wherein the low-G outlet passage (74) opens into the separation channel (66b) at the upstream end (67) of the separation channel (66b).

14. The fluid separation chamber (32) of any one of the preceding claims, wherein the high-G outlet passage (76) opens into the separation channel (66b) at the downstream end (69) of the separation channel (66b).

15. The fluid separation chamber (32) of any one of the preceding claims, wherein the low-G and high-G outlet passages (74, 76) open into the separation channel (66b) at a top end (63) of the separation channel (66b) and the inlet passage (68) preferably opens into the separation channel (66b) at a bottom end (62) of the separation channel (66b).
